# EUROPEAN PATENT SPECIFICATION

(11) **EP 4 234 062 B1**
(45) Date of publication and mention of the grant of the patent: **25.11.2026**
(21) Application number: 23181382.5
(22) Date of filing: 19.06.2017
(51) Int. Cl.: B01D 15/32, G01N 33/58, G01N 30/02, G01N 30/04, G01N 30/34, B01D 15/12, B01D 15/38, G01N 30/72

(54) **METHODS OF ELECTROSPRAY IONIZATION OF GLYCANS MODIFIED WITH AMPHIPATHIC, STRONGLY BASIC MOIETIES**
VERFAHREN ZUR ELEKTROSPRAYIONISATION VON MIT AMPHIPATHISCHEN, STARK BASISCHEN EINHEITEN MODIFIZIERTEN GLYKANEN
PROCÉDÉS D'IONISATION PAR ÉLECTRONÉBULISATION DE GLYCANES MODIFIÉS PAR DES FRAGMENTS AMPHIPATHIQUES, FORTEMENT BASIQUES

(30) Priority: 21.06.2016 US 201662352755 P
(43) Date of publication of application: 30.08.2023
(62) Document number(s) of the earlier application(s) in accordance with Art. 76 EPC: 17815988.5 / 3 472 621
(73) Proprietor: Waters Technologies Corporation, Milford, MA 01757 (US)
(72) Inventor: BROUSMICHE, Darryl W., Grafton, MA 01519 (US); LAUBER, Matthew A., North Smithfield, RI 02896 (US)
(74) Representative: Forresters IP LLP

(56) References cited:
- EP-A1- 1 750 126
- WO-A1-2016/069764
- MATTHEW A. LAUBER ET AL: "Rapid Preparation of Released N -Glycans for HILIC Analysis Using a Labeling Reagent that Facilitates Sensitive Fluorescence and ESI-MS Detection", ANALYTICAL CHEMISTRY, vol. 87, no. 10, 19 May 2015 (2015-05-19), pages 5401 - 5409, XP055214900, ISSN: 0003-2700, DOI: 10.1021/acs.analchem.5b00758
- MICHAEL KIMZEY ET AL: "Development of an Instant Glycan Labeling Dye for High Throughput Analysis by Mass Spectrometry", vol. V2-r2, 31 May 2015 (2015-05-31), pages 1 - 4, XP009525792, Retrieved from the Internet <URL:https://www.europa-bioproducts.com/productsheets/IPC_Glycan-Labeling-Dye-2015_handout_v3_r2.pdf>
- WATERS: "BEST PRACTICES IN THE ANALYSIS OF RAPIFLUOR-MS LABELED GLYCANS USING THE ACQUITY QDa DETECTOR (PERFORMANCE MODEL)", 1 March 2016 (2016-03-01), XP055659864, Retrieved from the Internet <URL:https://www.gimitec.com//file/720005655en.pdf> [retrieved on 20200121]

## Description

### BACKGROUND

When subject to electrospray ionization, labeled glycans in formic acid based mobile phases are prone to degradation via in-source fragmentation, even when relatively gentle mass spectrometry source conditions are employed and for glycans modified with strongly basic residues. A need exists, therefore, for higher sensitivity, less ambiguous MS analysis for these labeled glycans to be obtained.
WO2016/069764 provides methods of analyzing glycosylated biomolecules and EP 1750126 provides a method for analyzing compounds with amino group by LC/MS.

### SUMMARY OF THE INVENTION

Provided herein are methods and systems for electrospray ionization of glycans which have been modified with amphipathic, strongly basic moieties. A solution for use in electrospray ionization comprises a plurality of glycans having an amphipathic moiety and a basic residue of pKa >5, and one or more volatile components selected from the group consisting of an amine, ammonia, ammonia salt, diethylamine, or trimethylamine. In an aspect, the solution has a pH between about 3 to about 6, and ionic strength of between about 0 mM to about 500 mM. In an aspect, the solution can further comprise a solvent. In an aspect, the solvent can be acetonitrile, methanol, tetrahydrofuran, ethanol or isopropyl alcohol. Furthermore, in an aspect, the glycan is modified with an amphipathic, strongly basic moiety has a Log P value between about 1 and about 3. In an aspect, the amphipathic, strongly basic moiety has a pKa value greater than 6. In an aspect, the basic residue is a tertiary amine. In an aspect, the amphipathic moiety of the glycan is imparted by RFMS. In an aspect, the glycans can be an 0-glycan or an N-glycan.

Also, provided herein are chromatographic systems for glycan analysis that include a solution having a plurality of glycans modified to include an amphipathic moiety and basic residue of pKa >5, and one or more volatile components selected from the group consisting of an amine, ammonia, ammonia salt, diethylamine, or trimethylamine, and a chromatographic device comprising a hydrophobic stationary phase. In an aspect, the solution has a pH between about 3 to about 6, and ionic strength of between about 0 mM to about 500 mM. In an aspect, the chromatographic system has a reversed-phase retention mechanism. In an aspect, the chromatographic device is a combination of reversed-phase chromatography and mixed mode reversed-phase chromatography. In an aspect, the glycans can be an 0-glycan or an N-glycan.

Further provided herein are methods of detecting a glycan comprising the steps of: (1) providing a sample having a glycan; (2) forming a derivatized glycan in the sample wherein the glycan is modified to include an amphipathic moiety and basic residue of pKa greater than 5; (3) separating the derivativized glycans in a chromatographic device and in a solution comprising one or more volatile components selected from the group consisting of an amine, ammonia, ammonia salt, diethylamine, or trimethylamine, the solution having a pH between about 3 to about 6, and ionic strength of between about 0 mM to about 500 mM; and (4) detecting the separated derivatized glycan using electrospray ionization. In an aspect, the glycans can be an O-glycan or an N-glycan.

### BRIEF DESCRIPTION OF THE DRAWINGS

Figure 1 shows an exemplary chemical structure of a glycan modified with an amphipathic, strongly basic moiety.
Figure 2 shows yet another exemplary chemical structure of a glycan modified with an amphipathic, strongly basic moiety.
Figure 3A is an ESI mass spectrum for RFMS labeled FA2G2 glycan as electrosprayed from a solution comprising 0.1 percent formic acid in 20 percent acetonitrile.
Figure 3B is an ESI mass spectrum for a labeled FA2G2 glycan as electrosprayed from a solution comprising approximately 20:80 acetonitrile and 50 mM ammonium formate having a pH of 4.4.
Figure 3C is an ESI mass spectrum for 2-AB labeled FA2G2 glycan as electrosprayed from strongly acidic ESI+ conditions comprising 0.1 percent formic acid in 20 percent acetonitrile and stabilized by solutions buffered with volatile ammonium salts between pH 3 and pH 6.
Figure 3D is an ESI mass spectrum for the 2-AB labeled FA2G2 glycan as electrosprayed from a solution comprising approximately 20:80 acetonitrile and 50 mM ammonium formate having a pH of 4.4.

### DETAILED DESCRIPTION

Glycans are found throughout biological systems in both a free state as well as in conjugated forms as parts of glycoproteins, glycolipids and proteoglycans. Glycans play a role in a variety of biological and physiological process. Furthermore, the structures of glycans are diverse and complex. As a result, analysis of glycan profiles can be difficult.

With recent advancements in mass spectrometry instrumentation, the combination of liquid chromatography, fluorescence and MS has gained popularity as an analytical instrument platform for routine characterization of fluorescently labeled N-linked glycans. Relative quantitation and molecular weight measurements can be done in a single analysis. Shigeo Suzuki et al., Comparison of the Sensitivities of Various Derivatives of Oligosaccharides in LC/MS with Fast Atom Bombardment and Electrospray Ionization Interfaces, 1006 ANAL CHEM 2073 (1996).

High performance liquid chromatography ("HPLC") can be used to analyze glycan profiles. These methods include, but are not limited to, reversed phase chromatography, ion exchange chromatography and hydrophilic interaction chromatography ("HILIC") separations. Released glycans can be separated by HILIC or, alternatively, reversed phase chromatography, using graphitic stationary phases such as porous graphitized carbon and mobile phases acidified by formic acid. In addition, glycans can be separated by capillary electrophoresis.

Upon separation, the detection process can be carried out using either an absorbance or fluorescence detector. As each labeled glycan is eluted from the chromatographic column after separation, its presence and quantity is detected by a mass spectrometer and/or by its optical properties. The sensitivity of the assay, of course, depends upon the strength of the signal produced. Methods for analyzing glycans have become considerably sophisticated. Exemplary analytical instrumentation and associated chromatographic devices that can analyze glycans include, but is not limited to, capillary electrophoresis ("CE"), high-performance anion exchange chromatography coupled with electrochemical detection ("HPAEC-PED"), hydrophilic interaction chromatography with fluorescence detection ("HILIC-LC/FLR"), reversed-phase liquid chromatography coupled with mass spectrometry ("RPLC/MS"), and matrix-assisted laser desorption/ionization mass spectrometry ("MALDI-MS.")

Notwithstanding, glycans do not ionize efficiently *via* electrospray ionization ("ESP") and are not readily detectable by liquid chromatography mass spectrometry ("LC-MS") alone because absorbance and fluorescence responses are quite weak. Even larger glycans, such as heavily sialylated and polysiaylated glycans, are not detected because they are extremely labile and exhibit low ionization efficiency.

Therefore, in order to increase detection sensitivity, a glycan can be converted into a derivative glycan (also referred to herein as a "labeled glycan") with a derivatization reagent. The derivatizing reagent can affect the sensitivity and accuracy of the analysis by maximizing the sensitivity, yield and stability of the derivatized glycans. As such, certain derivation reagents (also referred to herein as a "reagent" or "labeling reagent") have been shown to form stable, highly fluorescent MS derivative compounds and conjugate glycans. Selection of the labeling reagent is central to an analytical procedure.

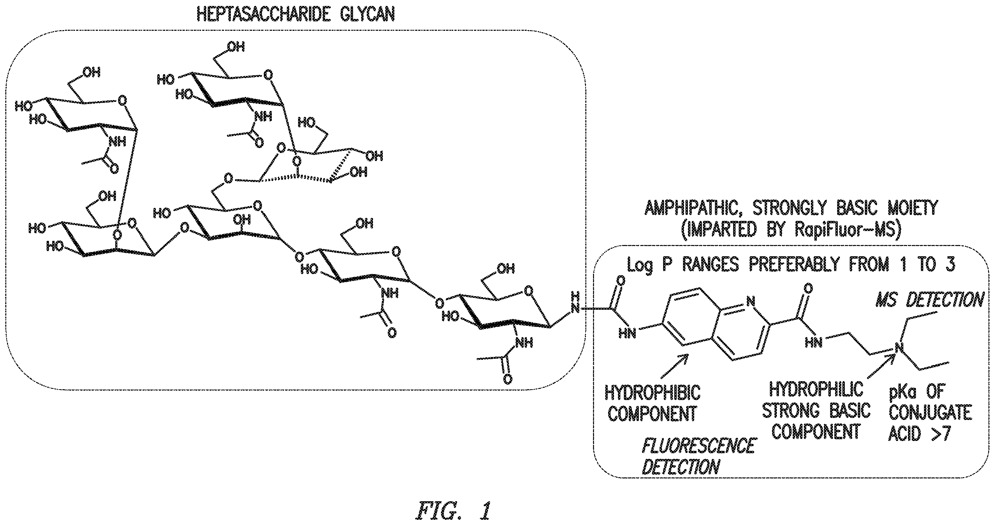

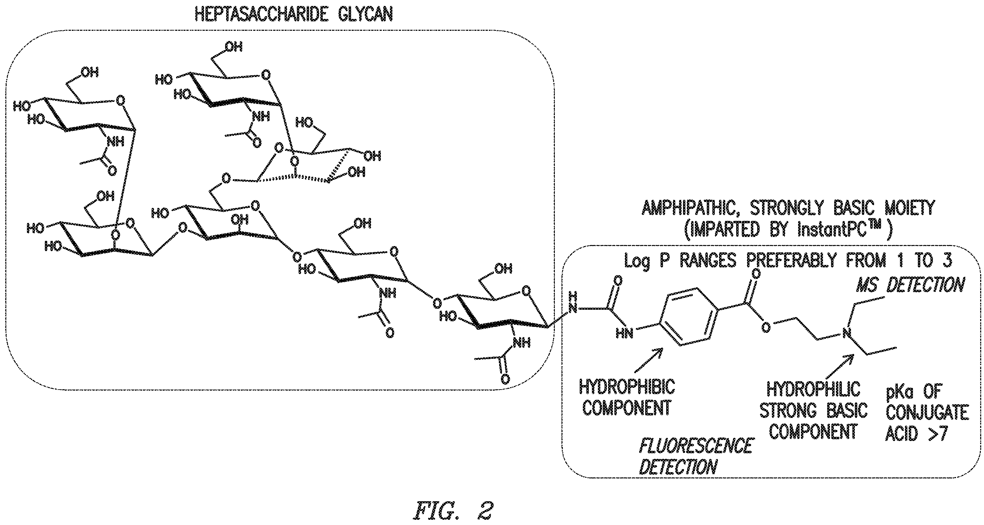

For example, recent work from our laboratory has demonstrated that a novel glycosylamine labeling reagent, RapiFluor-MS ("RFMS") having the chemical structure: 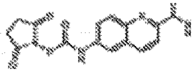 can form a labeled glycan ("RFMS-labeled glycan") having enhanced MS sensitivity during HILIC analyses based on positive ion mode electrospray ionization ("ESI+"). Lauber, M. A. et al, Rapid Preparation of Released N-Glycans for HILIC Analysis Using a Labeling Reagent that Facilitates Sensitive Fluorescence and ESI-MS Detection, Anal Chem, 87 (10), 5401-9 (2015). Figures 1 and 2 show heptasaccharide glycan portion labeled with RFMS, such that it is modified with an amphipathic, strongly basic moiety. As described, these particular reagents (RFMS and InstantPC) incorporates an amphipathic label onto glycans that increases analyte hydrophobicity and introduces a basic residue (pKa>5), a tertiary amine. These characteristics delineate the structure of the labeled glycan that displays gas phase proton affinity. For example, RFMS-labeled glycans can show high ionization efficiencies during ESI+MS as well as correspondingly high charge state (>2+), protonated ions.

The glycan label imparted by the RFMS reagent (FIG. 1) represents only one example of an amphipathic, strongly basic moiety. Other reagents having corresponding labeling moieties that with the same characteristics can be used, including, but not limited to the labeling reagents identified in US patent application number 14/458,760 entitled *Rapid Fluorescence Tagging of Glycans and Other Biomolecules with Enhanced MS Signals,* pages 2, lines 4 to page 4, line 9; page 11 line 4 to page 25, line 18 and page 29, line 1 to page 30 line 10 ; US Pat. No. 7,148,069, entitled *Method for Analysis of Compounds With Amino Group and Analytical Reagent Therefor,* at Col. 8, 1. 56 to Col. 9, 1. 54 and Col. 15, 1. 22 to 29; US Pat No. 7,494,815 entitled *Method and Apparatus for Analyzing Compounds with Amino Group,* at Col. 7,1. 19 to Col. 11,1. 24; US Pat. No. 8, 124,792, at Col. 2, 1. 13 to Col. 4, 1. 5 and Col. 7, 1. 11 to Col. 17, 1. 20 ; and US Pat. No. 5,296,599 entitled *Activated Carbamates Compounds* at Col. 4,1. 66 to Col. 5,1. 32 and Col. 5,1. 66 to Col. 7,1. 28. Each of the reagents contains amphipathic, strongly basic moieties with log P values between 0 and 5, preferably between 1 and 3, and pKa values greater than 6, preferably greater than 8. Additional exemplary reagents can contain procainamide and procaine moieties incorporated into a glycan through a secondary amine, urea, or amide linkage. *See,* U.S. Provisional Patent Application Nos. 62/352,724 and 62/352,734. The term moiety as used herein means a chemical functional group or plurality of chemical functional groups, including but not limited to a quinoline ring or guinoline ring combined with diethylaminoethyl group. Additional exemplary moieties useful in connection with the present methods and systems are described in Application No. PCT/US2012/057996 published as WO 2013/049622, at page 21, line 9 through page 30, line 10; US Pub. No. 2016/0139136, page at 2, line 9 through page 4, line 8 (referenced in Formula I as "FL-R³)," .

Glycans labeled with the reagents having amphipathic, strongly basic moieties can be readily amenable to HILIC (as well as ESI+MS) when conventional HILIC buffers such as ammonium formate buffer titrated to pH 4.4, are used for the mobile phases. Notwithstanding, unexpected problems have been faced in utilizing labeled glycans in reversed phase liquid chromatography ("RPLC") under certain conventional RPLC electrospray conditions. For example, sometimes labeled glycans are strongly retained and insufficiently resolved. Therefore, described herein are methods and systems having solution conditions that can be used to facilitate the detection of labeled glycans during separations based on reversed phase liquid chromatography, specifically detection by ESI-MS analysis of glycans labeled with amphiphatic strongly basic moieties, wherein glycans are electrosprayed from a solution having pH between 2.8 and 7, or about 3 and about 6.

General methodology for an efficient and effective analysis of the derivatized glycan includes three closely related processes: (1) formation of derivatized glycan in the sample; (2) separation of the derivatized glycans; and (3) detection of the separated derivatized glycans. Formation of the derivatized glycan is performed through one of several chemical reactions by reacting a biological sample containing the glycan (or glycan having been already separated from the biological sample) with one or more reagents to yield a derivatized glycan (sometimes referred to herein as a "derivatized compound," "derivatized glycan," "labeled glycan," "RFMS-labeled glycan" or "labeled compound"). Different processes for forming the derivatized glycan include, but are not limited to, rapid tagging or reductive amination. Such processes are described in PCT/2015/057848, published as WO 2016/069664 and particularly as summarized at pages 2 and 4. Formation of an effective labeled glycan, however, is only the first step.

Separation processing of the labeled glycan is based upon the differences in the chemical structure of the derivatized glycan. See e.g., Qing, Y. et al., A Rapid Sample Preparation Method for Mass Spectrometric Characterization of N-Linked Glycans, Rapid Commun. Mass Spectrom 2005 19 2331-2336 (2008). Labeled glycans can differ from each other in the same way that the chemical structures of the precursor compounds differ. In the present methods, the labeled glycan is separated so that the detector signal can be correctly related to the concentration of each glycan. As noted above, in these methods, the labeled glycan is separated chromatographically either solely or partially by a reversed-phase retention mechanism and subsequently detected by electrospray ionization.

Generally, in-source fragmentation ("ISF") is directly related with the desolvation and activation energy at the interface of electrospray ionization ("ESP") source. The desolvation/activation energy is controlled by the cone voltage (also called nozzle-skimmer voltage, or declustering voltage). Higher desolvation/activation energy can enhance ion yield but simultaneously facilitate ISF due to elevated ion energy, which compromises the final ion yield. In practice, researchers can optimize the ESI-MS desolvation process by balancing these two inversely proportional factors to maximize ion gain.

When not separated by HILIC, glycans released from a biological mixture are often separated by reversed-phase liquid chromatography and other chromatographic methods using a chromatographic device. For reversed-phase chromatography, the chromatographic devices can be graphitic stationary phases, including but not limited to, porous graphitized carbon, and strongly acidic mobile phases that are typically based only on formic acid. West, C. et al., Porous Graphitic Carbon: a Versatile Stationary Phase for Liquid Chromatography, J Chromatogr A 1217 (19), 3201-16 (2010); Ruhaak, L. R., Glycan Labeling Strategies and Their Use in Identification and Quantification, Anal Bioanal Chem 397 (8), 3457-81 (2010). When subject to electrospray ionization in formic acid based mobile phases, however, labeled glycans modified with strongly basic residues are prone to degradation via in-source fragmentation even when relatively gentle MS source conditions are employed.

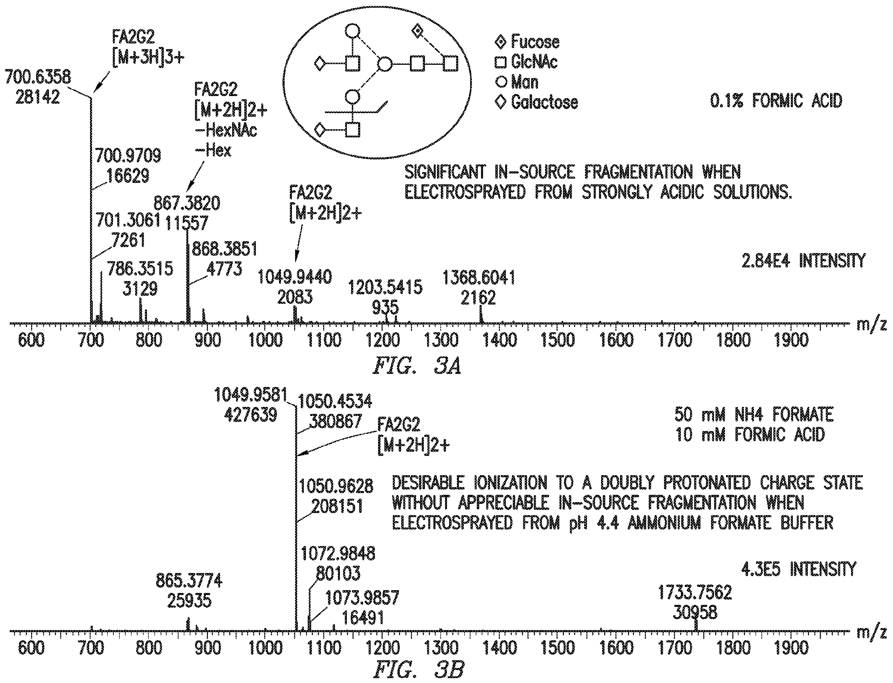

Figures 3A and 3B show that RFMS-labeled N-glycans can be labile when electrosprayed from strongly acidic ESI+ conditions but stabilized by solutions buffered with volatile ammonium salts between pH 3 and 6. By way of comparison, in Figure 3A, an ESI mass spectrum is shown for a RFMS-labeled FA2G2 glycan electrosprayed from a solution consisting of approximately 0.1% formic acid in 20% acetonitrile. In Figure 3B, the ESI mass spectrum is shown for a RFMS-labeled FA2G2 glycan electrosprayed from a solution comprising approximately 20:80 acetonitrile:50 mM ammonium formate pH 4.4. In these figures, "-HexNAc" denotes a loss of a N-acetylated hexosamine residue. "-Hex" denotes a loss of a hexose residue. Glycans are named according to the Oxford notation.

Figure 3A demonstrates a mass spectrum for RFMS-labeled FA2G2 obtained by electrospraying glycans out of a solution comprising approximately 0.1% formic acid in 20% acetonitrile. Significant in-source fragmentation occurred. Notice that charging occurred up to a triply protonated charge state. More problematically, the spectrum shows significant levels of ions resulting from in-source fragmentation. This has been found to occur even with the use of source potentials and temperatures comparatively lower than source parameters previously published as optimal conditions for RFMS-labeled N-glycans. *See e.g.,* Lauber, M. A.; et al., Rapid Preparation of Released N-Glycans for HILIC Analysis Using a Labeling Reagent that Facilitates Sensitive Fluorescence and ESI-MS Detection, Anal Chem 2015, 87 (10), 5401-9, at 5407to 5408 . Comparatively, a higher pH solution stabilizes the labeled glycan against ISF. As shown in Figure 3B, ESI mass spectrum for a RFMS-labeled FA2G2 glycan electrosprayed from a solution consisting of approximately 20:80 acetonitrile:50 mM ammonium formate pH 4.4. This mass spectrum shows desirable ionization to a doubly protonated charge state without appreciable in-source fragmentation when electrosprayed from pH 4.4 ammonium formate buffer.

As described herein, the present methodologies provide solution conditions that can be used to facilitate the liquid chromatography mass spectrometry ("LC-MS") analysis of glycans modified with amphipathic, strongly basic moieties. RFMS-labeled FA2G2 has been found to show a much higher signal-to-noise, lower background mass spectrum when electrosprayed from a solution comprising approximately 20:80 acetonitrile: 50 mM ammonium formate/10 mM formic acid pH 4.4. These effects are counter to previous observations for glycans labeled with a conventional fluorophore label, such as 2 aminobenzamide ("2-AB"). *See,* Figures 3A and 3B.

Glycans labeled with amphipathic, strongly basic residues can be be forced to inordinately high charge states and low m/z values when electrosprayed out of strongly acidic solutions (pH ≤3). It appears that the high charge state, low m/z species is inherently unstable and prone to in-source fragmentation. This problem can be avoided through the use of solution conditions described herein.

As such upon investigating this phenomenon, we have discovered that glycans labeled with amphipathic, strongly basic residues are optimally electrosprayed using pH 3 to pH 6 solutions that have been buffered with volatile components, including but not limited to ammonium formate, formic acid, ammonium acetate, and/or acetic acid. Use of a volatile pH 3 to pH 6 buffer during the LC-ESI+MS analyses of glycans, especially those glycans labeled with amphipathic, strongly basic residues, can lead to stabilization of the labeled glycan. The solutions and solution conditions provided herein are novel for reversed-phase LC-MS and direct infusion MS of glycans labeled with amphipathic, strongly basic residues. Glycans labeled with amphipathic, strongly basic residues have been shown to have a propensity to undergo in-source fragmentation. The solutions taught herein can be combined with more than one liquid chromatography technique. For example, the solution conditions can be combined with conventional reversed phase chromatography and mixed mode reversed phase chromatography. Mixed mode chromatography can be typified by the combination of ion exchange retention with hydrophobicity-based retention mechanisms if reversed-phase liquid chromatography. Accordingly, stabilization of the labeled glycan during ESI is implemented when at least one mechanism of the separation process is based on a reversed-phase retention mechanism.

The term reversed-phase as used herein describes the chromatography mode that is just opposite of normal-phase HPLC. In normal-phase HPLC, the stationary phase is polar and retains compounds of the mobile phase having similar polarity. Compounds whose polarity is similar to that of the mobile phase will be preferentially attracted to it and move faster through the stationary phase. In this way, based upon differences in the relative attraction of each compound for each phase, a separation is created by changing the speeds of the analytes.

Stated differently, in normal phase chromatography, the stationary phase is hydrophilic and therefore has a strong affinity for hydrophilic molecules in the mobile phase. Thus, the hydrophilic molecules in the mobile phase tend to bind (or "adsorb") to the column, while the hydrophobic molecules pass through the column and are eluted first. In normal phase chromatography, hydrophilic molecules can be eluted from the column by increasing the polarity of the solution in the mobile phase.

Reversed-phase chromatography (also called "RPC," "reverse-phase chromatography," "reversed phase liquid chromatography," "RPLC" or "hydrophobic chromatography") is a chromatographic method that uses a hydrophobic stationary phase. RPC refers to liquid (rather than gas) chromatography. Historically, the introduction of a technique using alkyl chains covalently bonded to the solid support created a hydrophobic stationary phase or chromatographic device, which has a stronger affinity for hydrophobic compounds. The use of a hydrophobic stationary phase can be considered the opposite, or "reverse", of normal phase chromatography and therefore, the term "reversed-phase chromatography." Reversed-phase chromatography typically employs a polar (aqueous) initial mobile phase. As a result, hydrophobic molecules in the polar mobile phase tend to adsorb to the hydrophobic stationary phase, and hydrophilic molecules in the mobile phase will pass through the column and are eluted first. Hydrophobic molecules can be eluted from the column by decreasing the polarity of the mobile phase using an organic (non-polar) solvent, which reduces hydrophobic interactions. The more hydrophobic the molecule, the more strongly it will bind to the stationary phase, and the higher the concentration of organic solvent that will be required to elute the molecule. Mathematical and experimental considerations used in other chromatographic methods can also apply to reversed phase chromatography (for example, the separation resolution is dependent on the length of the column).

Theoretically, any inert non-polar substance or chromatographic device that achieves sufficient packing can be used for reversed-phase chromatography as the chromatographic device for reversed phase chromatography is a hydrophobic stationary phase. One such chromatographic device includes a column having an octadecyl carbon chain (C18)-bonded silica (USP classification L1). Other chromatographic devices include C8-bonded silica columns (L7 - 166 columns), pure silica columns (L3 - 88 columns), cyano-bonded silica columns (L10 73 columns) and phenyl-bonded silica columns (L11 - 72 columns). Note that C18, C8 and phenyl-bonded silica are dedicated reversed-phase resins, while cyano columns can be used in a reversed-phase mode depending on analyte and mobile phase conditions. Not all C18 columns have identical retention properties. Surface functionalization of silica can be performed in a monomeric or a polymeric reaction with different short-chain organosilanes used in a second step to cover remaining silanol groups (end-capping). While the overall retention mechanism remains the same, subtle differences in the surface chemistries of different stationary phases will lead to changes in selectivity. Columns can have different polarity. For example, PFP is pentafluorophenyl. CN is cyano. NH2 is amino. ODS is octadecyl or C18. ODCN is a mixed mode column of C18 and nitrile. SCX is strong cationic exchange substrate used for separation of organic amines. SAX is strong anionic exchange used for separation of carboxylic acid compounds.

In addition, mixtures of water or aqueous buffers and organic solvents can be utilized to elute analytes from a reversed-phase column. The solvents must be miscible with water, and the most common organic solvents used are acetonitrile, methanol, and tetrahydrofuran ("THF"). Other solvents can be used such as ethanol or 2-propanol (isopropyl alcohol). Elution can be performed isocratically where the water-solvent composition does not change during the separation process or by using a solution gradient (the water-solvent composition changes during the separation process, usually by decreasing the polarity). The pH of the mobile phase can have an important role on the retention of an analyte and can change the selectivity of certain analytes. Charged analytes can be separated on a reversed-phase column by the use of ion-pairing (also called ion-interaction). This technique is known as reversed-phase ion-pairing chromatography.

With reversed-phase stationary phases bonded with C18, separations tend to produce problematic co-elutions, given that there is often poor selectivity among glycan structures differing with respect to net charge. N-glycans that have been released from glycoproteins are quite different unto themselves. In particular, released N-glycans exhibit different charge characteristics than peptides, as they will generally contain only neutral or acidic hydrophilic residues. The acidic residues in released N-glycans, and their carboxylic acids or phospho-groups, significantly impact the characteristic and net charge state of glycan species. Glycans containing more acidic residues will have greater negative net charge. Similarly, these acidic species are often correlated with the efficacy of a biopharmaceutical.

Furthermore, chromatographic devices such as those described in US Patent Pub. No. 2013/03199086, provide multimodal chromatographic media (retention mechanism) and methods of analyzing glycans that provide high resolution between different biological macromolecules, unique selectivity based on size, composition, structure (e.g., isomerism, linkages) and/or charge. These types of columns allow the macromolecules eluted from the media to be detected by standard methodology (e.g., mass spectrometry, fluorescence detection) with no, or minimal, clean up or purification post-analysis and pre-detection (e.g., fluorescent
tagging). Furthermore, to achieve sufficient sensitivity and selectivity for the complete separation of glycans, particularly N-glycans, chromatographic devices having high purity chromatographic materials ("HPCMs") comprising a chromatographic surface can be used. Here, the chromatographic surface (retention mechanism) has a hydrophobic surface group and one or more ionizable modifiers. Such HPCMs are described in US Patent Application Number 62/326,783 filed April 24, 2016 unpublished, pages 5 and 6.

In the present methods, the solution can be buffered with ammonium formate, formic acid, ammonium acetate and/or acetic acid and the like. The ionic strength of the mobile phase at which glycans elute and are electrosprayed can range from infinitely low and near 0 mM up to about 500 mM, and between about 1 and about 100 mM. This phenomenon can be attributable to a counter ion than can pair with glycan residues. In that way, an aspect of the methodologies presented herein is the use of a volatile component, such as an amine compound at pH 3 to 6 conditions. Likewise, while the separation of labeled glycans is described herein in an acetonitrile/water mobile phase systems, other solvents can be utilized and paired together in mobile phase systems, including but not limited to, methanol, isopropanol, butanol, n-propanol, and tetrahydrofuran. The volatile component is an amine, including but not limited to, ammonia, diethylamine, or triethylamine. In an aspect, the extreme charging that is evident in the mass spectra of a RFMS-labeled glycan electrosprayed out of strongly acidic conditions could be purposely exploited for analytical reasons, for instance to glean compositional or structural information. This aspect can be used in connection with any glycan labeled with an amphipathic, strongly basic moiety.

### EXAMPLE I

### Electrospray Ionization Mass Spectrometry (ESI-MS) of Glycans Labeled with RapiFluor-MS versus 2-AB

RFMS and 2-AB labeled N-glycans were prepared from human IgG (Sigma I4506) according to previously published conditions. *See e.g.,* Lauber, M. A.; Yu, Y. Q.; Brousmiche, D. W.; Hua, Z.; Koza, S. M.; Magnelli, P.; Guthrie, E.; Taron, C. H.; Fountain, K. J., Rapid Preparation of Released N-Glycans for HILIC Analysis Using a Labeling Reagent that Facilitates Sensitive Fluorescence and ESI-MS Detection, Anal Chem 87 (10), 5401-9 (2015).

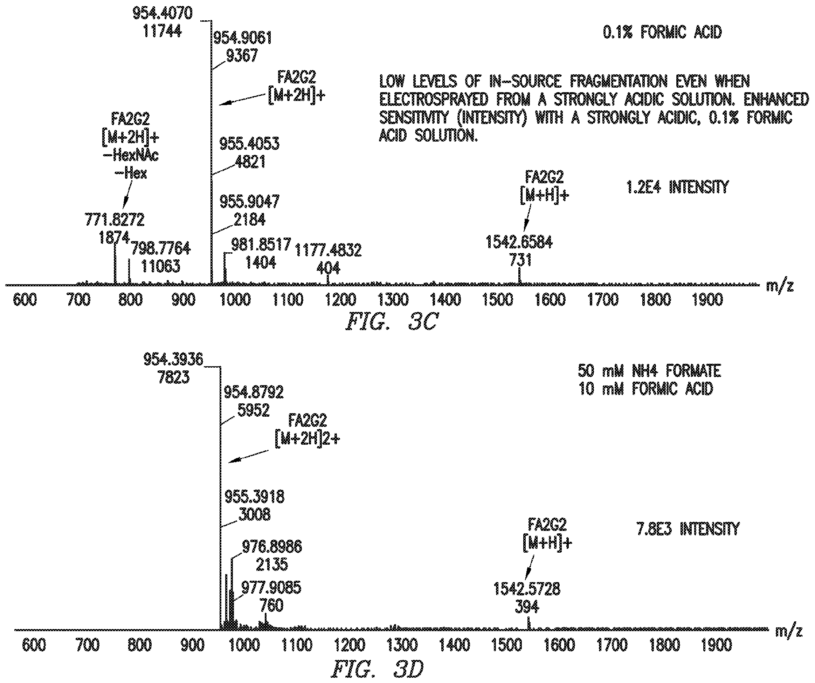

The labeled FA2G2 glycans were then electrosprayed at a flow rate of 200 µL/min from a solution consisting of approximately 0.1% formic acid in 20% acetonitrile or a solution consisting of approximately 80:20 acetonitrile:50 mM ammonium formate pH 4.4. Mass spectra were acquired using a Waters Synapt G2-S mass spectrometer. Comparatively gentle MS conditions were employed in these analyses that contrast the differences between electrospray conditions as shown in Figures 3A, 3B, 3C and 3D.

Immediately below were the mass spectrometry conditions utilized:

| Polarity | ES+ |
|---|---|
| Acquisition: | 700-2000 m/z (1 Hz) |
| Capillary (kV) | 3 |
| Source Temperature (°C) | 100 |
| Sampling Cone | 30 |
| Source Offset | 50 |
| Desolvation Temperature (°C) | 300 |
| Desolvation Gas Flow (L/Hr) | 800 |
| Nebuliser Gas Flow (Bar) | 7.0 |

## Claims

1. A chromatographic system for glycan analysis comprising:
a solution having a plurality of glycans modified to include an amphipathic, strongly basic moiety having a basic residue of pKa >5, the solution buffered with volatile components including ammonium formate and/or formic acid, and the solution having a pH between 3 to 6, and an ionic strength of between 1 mM to 500 mM; and
a chromatographic device comprising a hydrophobic stationary phase, the chromatographic device comprising a reversed-phase retention mechanism.

2. The chromatographic system of claim 1 wherein the plurality of glycans are N-glycans and/or O-glycans.

3. The chromatographic system for glycan analysis of claim 1, wherein the chromatographic device is a combination of reversed-phase chromatography and mixed mode reversed-phase chromatography.

4. A method of detecting a glycan comprising the steps of:
providing a sample wherein the sample comprises a glycan;
forming a derivatized glycan in the sample wherein the glycan is modified to include an amphipathic, strongly basic moiety having a basic residue of pKa >5;
separating the derivatized glycans in a reversed-phase chromatography method using a chromatographic device and in a solution buffered with volatile components including ammonium formate and/or formic acid, the solution having a pH between 3 to 6, and an ionic strength of between 1 mM to 500 mM; and
detecting the separated derivatized glycan using electrospray ionization.

5. The method of claim 4, wherein the glycan is an O-glycan or an N-glycan.

6. The method of claim 4, wherein the basic residue is a tertiary amine.

## Patentansprüche

1. Chromatographiesystem zur Glykananalyse, umfassend:
eine Lösung mit einer Vielzahl von Glykanen, die so modifiziert sind, dass sie einen amphipathischen, stark basischen Anteil mit einem basischen Rest von pKa > 5 umfassen, wobei die Lösung mit flüchtigen Komponenten gepuffert ist, die Ammoniumformiat und/oder Ameisensäure umfassen, und die Lösung einen pH-Wert zwischen 3 und 6 sowie eine Ionenstärke zwischen 1 mM und 500 mM aufweist; und
eine Chromatographievorrichtung, umfassend eine hydrophobe stationäre Phase, wobei die Chromatographievorrichtung einen Umkehrphasen-Retentionsmechanismus aufweist.

2. Chromatographiesystem nach Anspruch 1, wobei die Vielzahl von Glykanen aus N-Glykanen und/oder O-Glykanen besteht.

3. Chromatographiesystem zur Glykananalyse nach Anspruch 1, wobei die Chromatographievorrichtung eine Kombination aus Umkehrphasenchromatographie und Mischmodus-Umkehrphasenchromatographie aufweist.

4. Verfahren zum Nachweis eines Glykans, umfassend die Schritte zum: Bereitstellen einer Probe, wobei die Probe ein Glykan umfasst;
Bilden eines derivatisierten Glykans in der Probe, wobei das Glykan so modifiziert ist, dass es einen amphipathischen, stark basischen Anteil umfasst, der einen basischen Rest von pKa > 5 aufweist;
Abscheiden der derivatisierten Glykane in einem Umkehrphasenchromatographie-Verfahren unter Verwendung einer Chromatographievorrichtung und in einer Lösung, die mit flüchtigen Komponenten gepuffert ist, die Ammoniumformiat und/oder Ameisensäure umfassen, wobei die Lösung einen pH-Wert zwischen 3 und 6 sowie eine Ionenstärke zwischen 1 mM und 500 mM aufweist; und
Nachweis des abgeschiedenen, derivatisierten Glykans unter Verwendung einer Elektrospray-Ionisation.

5. Verfahren nach Anspruch 4, wobei das Glykan ein O-Glykan oder ein N-Glykan ist.

6. Verfahren nach Anspruch 4, wobei der basische Rest ein tertiäres Amin ist.

## Revendications

1. Système chromatographique pour l'analyse des glycanes comprenant :
une solution contenant une pluralité de glycanes modifiés pour inclure un fragment amphiphile fortement basique comportant un résidu basique dont le pKa est > 5, la solution étant tamponnée avec des composants volatils, notamment du formiate d'ammonium et/ou de l'acide formique, et la solution ayant un pH compris entre 3 et 6, et une force ionique comprise entre 1 mM et 500 mM ; et
un dispositif chromatographique comprenant une phase stationnaire hydrophobe, le dispositif chromatographique comprenant un mécanisme de rétention en phase inverse.

2. Système chromatographique selon la revendication 1, dans lequel la pluralité des glycanes sont des N-glycanes et/ou des O-glycanes.

3. Système chromatographique pour l'analyse des glycanes selon la revendication 1, dans lequel le dispositif chromatographique est une combinaison de chromatographie en phase inverse et de chromatographie en phase inverse en mode mixte.

4. Procédé de détection d'un glycane comprenant les étapes suivantes :
fournir un échantillon, ledit échantillon comprenant un glycane ;
former, dans l'échantillon, un glycane dérivé, ledit glycane étant modifié pour inclure un fragment amphiphile fortement basique comprenant un résidu basique dont le pKa est > 5 ;
séparer les glycanes dérivés par un procédé de chromatographie en phase inverse à l'aide d'un dispositif chromatographique et dans une solution tamponnée comprenant des composants volatils, notamment du formiate d'ammonium et/ou de l'acide formique, la solution ayant un pH compris entre 3 et 6, et une force ionique comprise entre 1 mM et 500 mM ; et
détecter le glycane dérivé séparé au moyen d'une ionisation par électronébulisation

5. Procédé selon la revendication 4, dans lequel le glycane est un O-glycane et/ou un N-glycane.

6. Procédé selon la revendication 4, dans lequel le résidu basique est une amine tertiaire.
